(19) Europäisches Patentamt
European Patent Office
Office européen des brevets

(11) **EP 3 701 972 A2**

(12) **EUROPEAN PATENT APPLICATION**
published in accordance with Art. 153(4) EPC


(43) Date of publication:
**02.09.2020 Bulletin 2020/36**

(21) Application number: **18871728.4**

(22) Date of filing: **21.09.2018**

(51) Int Cl.:
***A61K 48/00*** *(2006.01)*

(86) International application number:
**PCT/KR2018/011248**

(87) International publication number:
**WO 2019/083171 (02.05.2019 Gazette 2019/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.10.2017 KR 20170140190**
**20.09.2018 KR 20180112734**



(71) Applicant: **Curogene Life Sciences Co., Ltd**
**Heungdeok-gu**
**Cheongju-si, Chungcheongbuk-do**
**28424 (KR)**

(72) Inventors:
- **LEE, Young-Ill**
  **Cheongju-si**
  **Chungcheongbuk-do 28424 (KR)**
- **LEE, Steven Hyun Seung**
  **Cheongju-si**
  **Chungcheongbuk-do 28424 (KR)**
- **KIM, Yeryang**
  **Namyangju-si**
  **Gyeonggi-do 12273 (KR)**
- **CHOI, Jun-Sub**
  **Gyeonggi-do, 17027 (KR)**

(74) Representative: **Latscha Schöllhorn Partner AG**
**Grellingerstrasse 60**
**4052 Basel (CH)**


# (54) COMPOSITION COMPRISING RAAV CONTAINING SOLUBLE VEGFR-1 DERIVATIVE CDNA FOR TREATMENT OF MACULAR DEGENERATION


(57) The present invention relates to a pharmaceutical composition for treatment or prevention of macular degeneration and, more particularly, to a recombinant vector carrying a soluble VEGF receptor derivative cDNA and a pharmaceutical composition comprising the same for treatment or prevention of macular degeneration. According to the present invention, age-related macular degeneration, which is a representative retinal disease causing blindness in adults, can be effectively treated and a prophylactic effect can be expected.


Fig. 1

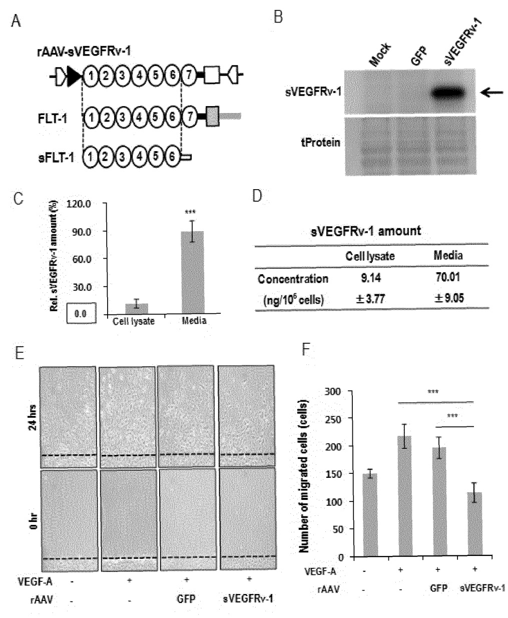


EP 3 701 972 A2

## Description

[Technical Field]

The present invention relates to a pharmaceutical composition for treating macular degeneration, and more particularly to a pharmaceutical composition comprising a recombinant vector containing a soluble VEGF-receptor-derivative cDNA.

[Background Art]

Macular degeneration is a disease that causes blindness by inducing degeneration of the retina, and is a serious age-related ophthalmic disease that can cause blindness. Macular degeneration is classified into wet macular degeneration and dry macular degeneration, and wet macular degeneration (wet AMD, age-related macular degeneration) refers to degeneration of the retinal macula associated with neovascularization, which is also called "neovascular macular degeneration". Meanwhile, macular degeneration not associated with neovascularization is dry macular degeneration (dry AMD) (Wong WL et al., Lancet Glob. Health; 2: e106, 2014).

In the case of dry macular degeneration, waste products form yellow deposits called "drusen" under the retina and the deposits are accumulated. A large amount of such accumulated deposits impedes the flow of blood to the retina, specifically the macula, obstructing vision and thus causing vision impairment. Dry macular degeneration does not cause rapid loss of vision, but may progress to wet macular degeneration.

Proteins accumulate between the retinal pigment epithelium (RPE) and the choroidal capillaries of the eye, causing yellow deposits called drusen to form and accumulate. The drusen impedes the migration of oxygen and nutrients from the choroidal capillaries, resulting in oxygen deficiency, impediment of material transport, and the incidence of inflammation, eventually causing the death of RPE cells and thus vision impairment.

There is currently no specific treatment for dry macular degeneration having this phenotype, and it is known that it is possible to slow down the progress to some extent using health foods having antioxidant effects such as vitamins, trace elements, and lutein. Recently, various clinical studies targeting complement-system-related proteins have been conducted, but all studies targeting C3, C5, and the like have failed, and lampalizumab, a monoclonal antibody drug developed for factor blockade (developed by Roche Corp.), exhibits efficacy, and phase III clinical trials thereon are underway.

Wet macular degeneration (wet AMD) occurs in 5-10% of patients suffering from dry macular degeneration, and is an acute disease that can cause blindness within a few months, when not treated, unlike dry macular degeneration, where the loss of vision progresses over a period of several years or even decades. In the case of wet macular degeneration, a wide range of oxygen partial pressure and nutrient deterioration, i.e., ischemia in tissues, and accompanying inflammatory reactions, oxidative stress, and complement system involvement, which plays an important role in the immunological mechanism across the subretinal space and subRPE space, cause choroidal neovascularization (CNV) characteristically occurring in the subretinal space or the subRPE space, leading to serous fluid leakage and bleeding (Nowak J.Z., Pharmacol. Rep. 58:353, 2006).

It is known that endothelial cells, RPE cells, and inflammatory cells, including monocytes, macrophages and the like, are involved in choroidal neovascularization (CNV). Treatment for wet macular degeneration has reduced the severity of blindness in many patients using VEGF antibodies since 2005. The reason for using these drugs is that VEGF is known to play a key role in choroidal neovascularization. However, the use of VEGF antibody does not completely inhibit the formation and growth of choroidal neovascularization. In particular, photoreceptor cells, which correspond to the central part of the retina where choroidal neovascularization occurs, eventually lose the function thereof over several years due to disintegration of the RPE tissues present thereunder. In addition, although the VEGF antibody is used, it acts only on endothelial cells present on the surface of new choroidal blood vessels, so the new choroidal blood vessels do not decrease in size, but rather continue to increase in size.

Macular degeneration is a disease caused by various pathogenic mechanisms such as environmental, genetic and aging-related factors. Thus, However treatment of macular degeneration with a protein-based drug has its limitations, and the main one being that currently commercially available drugs have a disadvantage of requiring intraocular injection initial every one or two months.

Meanwhile, gene therapy is a method of treating diseases through gene delivery and expression, and is a therapy that corrects genetic defects of specific target genes with disorders, unlike drug therapy. The ultimate goal of gene therapy is to obtain beneficial therapeutic effects through genetic modification of viable cells. Gene therapy is receiving attention as the best approach for disease treatment owing to the advantages of accurate delivery of genetic factors to disease sites, complete *in-vivo* degradation, absence of toxicity and immunogenicity, and long-term stable expression of genetic factors.

Gene therapy includes methods of introducing genes having therapeutic effects for specific diseases, enhancing the resistance function of normal cells to exhibit resistance to anticancer agents or the like, or replacing modified or deleted genes in patients of various genetic diseases and the like.

Gene therapy is largely divided into two types, namely *in-vivo* and *ex-vivo* gene therapy. *In-vivo* gene therapy includes injecting therapeutic genes directly into the body, and *ex-vivo* gene therapy includes primarily culturing target cells *in vitro,* introducing genes into the cells and then injecting the genetically modified cells into the body. Currently, the gene therapy research field uses *ex-vivo* gene therapy more than *in-vivo* gene therapy.

Gene transfer technologies may be broadly classified into a viral vector-based transfer method, a non-viral delivery method using synthetic phospholipids, synthetic cationic polymers or the like, and physical methods such as electroporation for introducing genes through temporary electrical stimulation of the cell membrane.

Among the gene delivery technologies, viruses used as virus transporters or virus vectors include RNA virus vectors (retrovirus vectors, lentiviral vectors, etc.), DNA virus vectors (adenovirus vectors, adeno-associated virus vectors, etc.), and the like. In addition, such viruses include herpes simplex viral vectors, vaccinia virus vectors, alpha viral vectors, and the like.

Meanwhile, adenovirus is a cloning vector having several advantages in that it can be replicated in a medium size in the cell nucleus, is clinically non-toxic, is stable even when an external gene is inserted, does not cause gene rearrangement or loss, can transform eukaryotic organisms, and is expressed stably at a high level even when integrated into a host cell chromosome. Suitable host cells for adenovirus are cells that cause human hematopoiesis, lymph and myeloma. However, adenovirus is linear DNA and thus is difficult to proliferate, is not easy to recover an infected virus, and has a low viral infection rate. In addition, the expression of the transferred gene is maximized after 1 to 2 weeks, and in some cells, expression is maintained for only 3 to 4 weeks. Also, there is a problem of high immunogenicity.

Adeno-associated virus (AAV) has recently been favored because it can overcome the problems described above and has many advantages as a gene therapeutic agent. AAV is a single-stranded provirus and requires an auxiliary virus for replication, and the AAV genome is 4, 680 bp in length and can be inserted into a specific region of chromosome 19 of the infected cell. The trans-gene is inserted into plasmid DNA linked by two 145 bp inverted terminal repeat (ITR) sequence parts and a signal sequence part. In order to produce an AAV-based gene therapeutic agent, additionally, another plasmid DNA expressing the AAV rep and cap parts is transfected therewith, and at the same time, adenovirus, which is absolutely necessary for AAV amplification, is directly added as a helper virus, or is added in the form of a separate plasmid containing the adenovirus genes (E1, E4, and VA RNA genes), which play an important role in amplification.

AAV has the advantages of a wide range of host cells delivering genes, fewer immunogenic side effects upon repeated administration, and maintenance of gene expression for a long period. Moreover, the AAV genome is safe even when integrated into the chromosome of the host cell and causes neither alteration nor rearrangement of the gene expression of the host.

Vascular endothelial growth factor (VEGF) binds to the fms-like tyrosine kinase receptor, Flt-1 and the pair of KDR/Flk-1, to act thereon, and the interaction with the latter causes an angiogenic process. Soluble Flt-1 (sFLT-1) is a soluble variant that lacks the membrane-spanning domain present in Flt-1 and is naturally generated through a selective splicing process. sFlt-1 binds directly to VEGF with high affinity to prevent VEGF from interacting with KDR/Flk-1 to exhibit anti-VEGF activity (anti-angiogenic activity). sFlt-1 also binds to KDR/Flk-1 itself to form an inactive heterodimer. Since this anti-VEGF activity functions as an anti-angiogenic activity, an antibody and fusion protein using anti-VEGF activity is currently used as the best therapeutic agent for macular degeneration, and such antibody and protein products include Lucentis® (ranibizumab), developed by Genentech, Inc. and marketed by Novartis AG, Eylea® (aflibercept) developed by Regeneron Pharmaceuticals, Inc., and Avastin® (bevacizumab), and the like. However, these protein therapeutic agents should be injected into the eyeball once every one to two months and cause serious side effects. Thus, there is a need for the development of a fundamental therapeutic agent for macular degeneration that is capable of exhibiting long-term treatment efficacy even when injected only a single time.

Accordingly, as a result of making efforts to develop novel therapeutic agents for macular degeneration that can exhibit therapeutic effects for several years through only a single administration, the present inventors developed a soluble VEGF receptor variant containing a new region rather than a naturally derived VEGF receptor, which has been studied and found that choroidal neovascularization was inhibited and the size of the lesion was reduced when a gene therapeutic agent packaged in the adeno-associated viral vector (AAV) was administered to a macular degeneration mouse model, and completed the present invention based on this finding.

[Disclosure]

[Technical Problem]

It is one object of the present invention to provide a composition for treating macular degeneration that can exhibit a therapeutic effect for several years even through a single administration.

[Technical Solution]

In accordance with one aspect of the present invention, the above and other objects can be accomplished by the provision of a recombinant vector containing a soluble VEGF receptor variant cDNA represented by SEQ ID NO: 1.

In another aspect of the present invention, provided is a pharmaceutical composition for treating or preventing macular degeneration comprising the recombinant vector comprising the soluble VEGF receptor variant cDNA (sVEGFRv-1 cDNA).

In another aspect of the present invention, provided is a method of treating or preventing macular degeneration comprising the recombinant vector comprising the soluble VEGF receptor variant cDNA (sVEGFRv-1 cDNA).

In another aspect of the present invention, provided is use of the recombinant vector comprising the soluble VEGF receptor variant cDNA (sVEGFRv-1 cDNA) for the treatment or prevention of macular degeneration.

In another aspect of the present invention, provided is use of the recombinant vector comprising the soluble VEGF receptor variant cDNA (sVEGFRv-1 cDNA) for the preparation of a drug for treating or preventing macular degeneration.

[Description of Drawings]

FIG. 1A compares the configuration of a soluble VEGF receptor variant inserted into rAAV2-sVEGFRv-1 used in the present invention with that of sFlt-1, a naturally derived soluble VEGF receptor.

FIGS. 1B to 1D show the results of Western blotting (FIG. 1B) and ELISA (FIGS. 1C and 1D) analysis identifying that sVEGFRv-1 protein is secreted from the cell into the medium and expressed in a soluble form in HeLa cells treated with rAAV2-sVEGFRv-1 or rAAV2-GFP.

FIGS. 1E and 1F show the results of a migration assay of human umbilical vein endothelial cells (HUVEC) treated with rAAV2-sVEGFRv-1 or rAAV2-GFP.

FIGS. 2A and 2C show the results of RT-PCR confirming the expression of sVEGFRv-1 or GFP in human retinal pigment epithelial cells (ARPE-19 cells) treated with rAAV2-sVEGFRv-1 or rAAV2-GFP, and FIGS. 2B and 2D show the results of RT-PCR confirming the expression of VEGFRv-1 or GFP in the eye tissue of rats treated with rAAV2-sVEGFRv-1 or rAAV2-GFP.

FIG. 3 shows a whole mount immunostained with phalloidin (left) and anti-CD31 (middle) to evaluate anti-angiogenic efficacy against neovascularization in a mouse CNV model, a macular degeneration model having choroidal neovascularization (CNV) induced through laser irradiation for untreated groups (A-C), rAAV2-GFP treated-groups (D-F), rAAV2-sVEGFRv-1-treated groups (G-I), and bevacizumab-treated groups (J-L), wherein M shows the result of statistical analysis of CNV size compared with the untreated group (n = 5, **; $P < 0.01$) and "Beva" represents a bevacizumab-treated group.

FIG. 4 shows the result of immunohistochemical analysis of choroidal infiltration of inflammatory cells using anti-F4/80 for untreated groups (A-C), rAAV2-GFP-treated groups (D-F), rAAV2-sVEGFRv-1-treated groups (G-I), and bevacizumab-treated groups (J-L), wherein "Beva" represents a bevacizumab-treated group. M shows the result of statistical analysis of staining degree of anti-F4/80, compared with the untreated group (n = 5, **; $P < 0.01$), and "Beva" represents a bevacizumab-treated group.

FIG. 5 shows the result of immunohistochemical analysis of infiltration and fibrosis of inflammatory cells based on CD11b and pan cytokeratin expression, respectively, for untreated groups (A-D), rAAV2-GFP-treated groups (E-H), rAAV2-sVEGFRv-1-treated groups (I-L), and bevacizumab-treated groups (M-P), wherein "Beva" represents a bevacizumab-treated group. Q shows the result of statistical analysis of staining degree of anti-CDllb, compared with the untreated group (n = 5, **; $P < 0.01$), "Beva" represents a bevacizumab-treated group. R shows the result of statistical analysis of staining degree of pan cytokeratin, compared with the untreated group (n = 5, *; $P < 0.05$), and "Beva" represents a bevacizumab-treated group.

FIG. 6 shows TUNEL-positive cells for untreated groups (A-C), rAAV2-GFP-treated groups (D-F), rAAV2-sVEGFRv-1-treated groups (G-I), and bevacizumab-treated groups (J-L), wherein "Beva" represents a bevacizumab-treated group. M shows the result of statistical analysis of TUNEL-positive cells, compared with the untreated group (n = 5, **; P < 0.01), and "Beva" represents a bevacizumab-treated group.

[Best Mode]

Age-related macular degeneration is a blindness-inducing retinal disease that has increasing incidence with age, and the number of patients suffering therefrom increases every year due to environmental factors. Currently, the mechanism of treatment of neovascularization-associated wet macular degeneration is developed into the treatment through inhibition of VEGF-related pathogenesis, and most therapeutic agents are also linked to the mechanism of VEGF.

In the present invention, a therapeutic agent for delivering a therapeutic gene to the retina was developed by mounting a therapeutic gene on a recombinant adeno-associated virus (AAV) in order to suppress macular degeneration associated with neovascularization, and this was confirmed using an animal model.

In the present invention, in order to suppress macular degeneration caused by neovascularization, rAAV2-soluble VEGFRv-1 (rAAV2-sVEGFRv-1), which is a candidate for a soluble VEGFR-1 gene therapeutic agent, was developed to block the activity of VEGF in the retina by introducing a soluble VEGFR-1 variant therapeutic gene into a recombinant adeno-associated virus (AAV), and the therapeutic efficacy thereof was confirmed using CNV (choroidal neovascularization)-induced animal models. In addition, the rAAV2-soluble VEGFRv-1 gene therapeutic agent has therapeutic efficacy superior to that of the conventional antibody-based drug, bevacizumab.

Therefore, in one aspect, the present invention is directed to a recombinant vector comprising a soluble VEGF receptor variant cDNA represented by SEQ ID NO: 1.

In the present invention, the soluble VEGF receptor variant cDNA is a fragment of the spanning nucleotide position 282-2253 in the human vascular endothelial growth factor receptor (VEGFR) 1 gene (XM_017020485.1, NCBI reference Sequence, NIH) and has a total size of 1,972 bp (SEQ ID NO: 1).

In the present invention, the recombinant vector is preferably an adeno-associated virus, more preferably rAAV2 or another serotype of rAAV.

In one aspect, the present invention is directed to a composition for treating macular degeneration comprising the recombinant vector comprising the soluble VEGF receptor variant cDNA.

The soluble VEGF receptor variant cDNA used in the present invention is a fragment of the spanning nucleotide position 282-2253 in the human vascular endothelial growth factor receptor (VEGFR) 1 gene (XM_017020485.1, NCBI reference Sequence, NIH), which has a total size of 1,972 bp (SEQ ID NO: 1). As shown in FIG. 1A, the variant, which has the 6 immunoglobulin-like domains having binding sites with VEGF and PIGF and the same 31 N-terminals as FLT-1, and sFLT-1, which is naturally derived from FLT-1, has 6 immunoglobulin-like domains and a tail composed of 37 amino acids different from FLT-1, so the variants have different configurations from each other (Kendall, R.L. and Thomas, K.A. Proc. Natl. Acad. Sci. USA 90, 10705-10709, 1993).

In the present invention, the recombinant vector may be an adeno-associated virus, preferably rAAV2 or another rAAV serotype.

In one embodiment of the present invention, in order to produce rAAV2-sVEGFRv-1, the recombinant vector, including a soluble VEGF receptor variant (sVEGFRv-1) cDNA, sVEGFRv-1 cDNA was inserted into pAAV-F.IX cis plasmid (US Patent No. 6,093,392) containing a CMV promoter, an SV40 polyadenylation signal and two ITRs to produce pAAV-sVEGFRv-1, and all of pAAV-sVEGFRv-1, pAAV-R2C2, and pHelper were transfected into HEK293 (ATCC CRL-1573) cells, followed by culturing for 72 hours. Then, the HEK293 cells were collected and sonicated, and recombinant AAV (rAAV) particles were repeatedly subjected to CsCl density-gradient centrifugation twice, and portions having an RI (refractive index) of 1.37 to 1.42 g/mL upon the primary measurement and portions having an RI of 1.35 to 1.43 g/mL upon the secondary measurement were collected and purely isolated to obtain rAAV2-sVEGFRv-1 vector.

In another embodiment of the present invention, the anti-VEGF activity of rAAV2-sVEGFRv-1 produced above was confirmed. When VEGF was treated with HUVEC cells treated with rAAV2-sVEGFR-1, it was found that the cell migration of HUVEC cells in the rAAV2-sVEGFRv-1-treated group was decreased compared to the control group, the vector-untreated group, and the rAAV2-GFP-treated group, and the VEGF activity thereof was reduced.

In another embodiment of the present invention, the retinal section of the rAAV2-sVEGFRv-1-treated CNV mouse model was immunostained with anti-CD31 and phalloidin, and the intensity of the resulting choroidal neovascularization (CNV) was determined. As a result, damage to the RPE and formation of the penetrating new blood vessels were observed in untreated control mice and mice intravitreally injected with rAAV-GFP, whereas choroidal neovascularization (CNV) was significantly inhibited in the mice intravitreally injected with rAAV2-sVEGFRv-1.

In another embodiment of the present invention, it was identified that macrophages and leukocytes were reduced in the retinal section of the CNV mouse model treated with rAAV2-sVEGFRv-1, and thus inflammatory cell infiltration was reduced. This reduction was higher than that in the positive control group and bevacizumab-treated group, although the difference was not statistically significant.

In another embodiment of the present invention, as a result of immunostaining a transverse retinal section with anti-pan cytokeratin (ab27988; Abcam, San Francisco, CA) in order to identify the anti-fibrotic effect of rAAV2-sVEGFRv-1, the rAAV2-sVEGFRv-1-treated group (2534.7 ± 703.5, n=3) had a significantly reduced number of anti-pan cytokeratin-positive cells, similar to the bevacizumab-treated group (2507.0 ± 585.4, n = 3), which indicates an inhibitory effect on retinal fibrosis, which occurs characteristically in wet macular degeneration (AMD) (FIG. 5R).

In another embodiment of the present invention, TUNEL-positive cells were observed in order to confirm the apoptosis effect of rAAV2-sVEGFRv-1. The result showed that the number of TUNEL-positive cells was significantly lower in samples collected from mice treated with rAAV2-sVEGFRv-1, and this reduction of apoptosis was similar to the reduction of apoptosis achieved through bevacizumab treatment (FIG. 6M).

In another embodiment of the present invention, it was identified that, when rAAV2-sVEGFRv-1 was intravitreally injected into a macular degeneration CNV mouse model, efficacy was similar to that of bevacizumab, used as a positive control group, even when injected at a low concentration.

Therefore, the composition for treating macular degeneration with the present invention, as an AAV-based gene therapeutic agent that can overcome the problems of conventional therapeutic agents such as bevacizumab, including the trouble of repeated monthly intraocular injection, eye damage from injection, and side effects such, as inflammation and high treatment costs.

In the present invention, the anti-angiogenic, anti-inflammatory and anti-fibrotic effects of rAAV2-sVEGFR-1 were confirmed and thus rAAV2-sVEGFR-1 of the present invention was found to have therapeutic effects similar to those of bevacizumab, which is a known therapeutic agent for macular degeneration. In addition, rAAV2-sVEGFR-1 of the present invention has the advantage of exhibiting a continuous therapeutic effect for 2 to 3 years through a single injection, without the need for monthly direct intraocular injection, like bevacizumab.

In the present invention, the composition for treating macular degeneration may be prepared as an injection formulation and the injection formulation may further contain a stabilizer selected from the group consisting of ethyl oleate, polyvinylpyrrolidone (PVP10), ganglioside sodium L-lactate, zinc chloride, and sucrose. In a preferred embodiment, ethyl oleate 10 mg/ml, PVP10 1 mg/ml, GM1 0.1 mg/ml, sodium L-lactate 1 mg/ml, zinc chloride 0.1 mg/ml, and sucrose 10 mg/ml in HEPES buffer (20 mM, pH 7.4) may be used.

The carrier contained in the pharmaceutical composition according to the present invention may include a carrier, an adjuvant and a vehicle, which are collectively referred to as "pharmaceutically acceptable carrier". The pharmaceutically acceptable carrier that can be used for the pharmaceutical composition of the present invention may include, but are not limited to, ion exchangers, alumina, aluminum stearate, lecithin, serum proteins (*e.g.*, human serum albumin), buffer substances (*e.g.*, various phosphates, glycine, sorbic acid, potassium sorbate and partial glyceride mixtures of saturated vegetable fatty acids), water, salts or electrolytes (*e.g.*, protamine sulfate, disodium hydrogen phosphate, potassium hydrogen phosphate, sodium chloride and zinc salts), colloidal silica, magnesium trisilicate, polyvinyl pyrrolidone, cellulose-based substrates, polyethylene glycols, sodium carboxymethylcellulose, polyarylates, waxes, polyethylene-polyoxypropylene-blocking polymers, polyethylene glycols, wool fats, and the like.

The therapeutic composition of the present invention is preferably administered parenterally. As used herein, the term "parenteral" includes subcutaneous, intradermal, intravenous, intramuscular, intraarticular, intrasynovial, intrasternal, intradural, intralesional, and intracranial injection or infusion techniques.

The pharmaceutical composition may be in the form of a sterile injectable preparation such as a sterile injectable aqueous or oily suspension. Such a suspension may be formulated according to techniques known in the art using a suitable dispersing or wetting agent (e.g., Tween 80) and a suspending agent. The sterile injectable preparation may also be a sterile injectable solution or suspension (*e.g.*, a solution in 1,3-butanediol) in a nontoxic parenterally acceptable diluent or solvent. Available acceptable vehicles and solvents may include mannitol, water, Ringer's solution, and isotonic sodium chloride solutions. In addition, sterile nonvolatile oils are commonly used as solvents or suspending media. For this purpose, any less stimulating nonvolatile oil containing synthetic mono-or di-glycerides may also be used. Fatty acids such as oleic acid and glyceride derivatives thereof are useful for injectable preparations, like pharmaceutically acceptable natural oils (*e.g.*, olive oil or castor oil), particularly polyoxyethylated forms thereof.

The composition of the present invention may be used in combination with a common anti-inflammatory agent or in combination with a matrix metalloprotease inhibitor, a lipoxygenase inhibitor, and an inhibitor of cytokine other than IL-1β. The composition of the present invention may be administered in combination with an immunomodulator (*e.g.*, bropirimine, anti-human alpha interferon antibody, IL-2, GM-CSF, methionine enkephalin, interferon-alpha, diethyldithiocarbamate, tumor necrosis factor, naltrexone and rEPO), or prostaglandin to prevent or eliminate symptoms of IL-1-mediated disease such as inflammation. When the composition of the present invention is administered in combination with other therapeutic agents, they may be administered to the patient sequentially or simultaneously. Alternatively, the pharmaceutical composition according to the present invention may be prepared by mixing the composition of the present invention with another therapeutic or prophylactic agent described above.

The amount of the antibody that can be combined with the carrier material to produce a single dosage form may vary depending on the host in need of treatment and on the particular mode of administration.

However, it will be understood that specific effective amounts for a particular patient may vary depending on several factors, including the activity of the particular compound that is used, age, weight, general health, gender, diet, administration time, route of administration, release rate, drug combination, and severity of the particular disease being prevented or treated. The pharmaceutical composition according to the present invention may be formulated into a pill, dragee, capsule, liquid, gel, syrup, slurry, or suspension.

In a preferred embodiment, for parenteral administration, the pharmaceutical composition of the present invention may be prepared as an aqueous solution. Preferably, a physically suitable buffer such as Hanks' solution, Ringer's solution, or physically buffered saline may be used. Aqueous injection suspensions may be added with a substrate that can increase the viscosity of the suspension, such as sodium carboxymethyl cellulose, sorbitol or dextran. In addition, the suspension of the active ingredient may be prepared as a suitable oily injection suspension. Suitable lipophilic solvents or carriers include fatty acids such as sesame oil or synthetic fatty acid esters such as ethyl oleate, triglycerides, or liposomes. Non-lipid polycationic amino polymers may also be used as carriers. Optionally, the suspension may use a suitable stabilizer or agent to increase the solubility of the compound and to prepare a highly concentrated solution.

In another aspect, the present invention is directed to a method of treating or preventing macular degeneration comprising the recombinant vector comprising the soluble VEGF receptor variant cDNA (sVEGFRv-1 cDNA).

In another aspect, the present invention is directed to the use of the recombinant vector comprising the soluble VEGF receptor variant cDNA (sVEGFRv-1 cDNA) for the treatment or prevention of macular degeneration.

In another aspect, the present invention is directed to the use of the recombinant vector comprising the soluble VEGF receptor variant cDNA (sVEGFRv-1 cDNA) for the preparation of a drug for treating or preventing macular degeneration.

As used herein, the term "treatment" refers to all actions that alleviate or cure the symptoms of the diseases by administration of a pharmaceutical composition comprising a recombinant vector comprising the soluble VEGF receptor variant cDNA (sVEGFRv-1 cDNA).

As used herein, the term "vector" means a DNA product containing a DNA sequence operably linked to a suitable control sequence capable of expressing DNA in a suitable host. The vector may be a plasmid, a phage particle, or a simple potential genome insert. Once the vector is transformed into an appropriate host, it may replicate and function independently of the genome of the host, or may often be integrated with the genome. Since the plasmid is the most commonly used type of vector, the terms "plasmid" and "vector" may be used interchangeably throughout the specification of the present invention. For the purpose of the present invention, a plasmid vector is preferably used. A typical plasmid vector that can be used for this purpose includes (a) a replication origin to efficiently conduct replication so as to include several hundred plasmid vectors in each host cell, (b) an antibiotic resistance gene to screen a host cell transformed with the plasmid vector, and (C) a restriction enzyme cleavage site into which a foreign DNA fragment is inserted. Even if an appropriate restriction enzyme cleavage site is not present, the vector and foreign DNA can be easily ligated using a synthetic oligonucleotide adapter or a linker according to a conventional method.

After ligation, the vector should be transformed into an appropriate host cell. In the present invention, the preferred host cells are prokaryotic cells. Suitable prokaryotic host cells include *E. coli* DH5α, *E. coli* JM101, *E. coli* K12, *E. coli* W3110, *E. coli* X1776, *E. coli* XL-1 Blue (Stratagene), *E. coli* B, *E. coli* B21, and the like. However, *E. coli* strains such as FMB101, NM522, NM538, and NM539, as well as species and genera of other prokaryotes and the like can also be used. In addition to *E. coli* mentioned above, strains of the genus Agrobacterium such as *Agrobacterium A4, Bacillus* strains such as *Bacillus subtilis,* other enterobacteria such as *Salmonella typhimurium* or *Serratia marcescens* and various Pseudomonas genus strains can be used as host cells.

Transformation of prokaryotic cells can be easily carried out using a calcium chloride method described in the section 1.82 of Sambrook et *al.,* supra. Alternatively, electroporation (Neumann, et al., EMBO J., 1: 841, 1982) can be used for transformation of these cells.

As used herein, the term "expression control sequence" means a DNA sequence essential for the expression of a coding sequence operably linked to a particular host organism. Such a control sequence includes promoters for conducting transcription, operator sequences for controlling such transcription, sequences for encoding suitable mRNA ribosome-binding sites, and sequences for controlling the termination of transcription and translation. For example, control sequences suitable for prokaryotes include promoters, optionally operator sequences and ribosome-binding sites. Eukaryotic cells include promoters, polyadenylation signals, and enhancers. The factor that has the greatest impact on the expression level of the gene in the plasmid is a promoter. SRα promoters, cytomegalovirus-derived promoters, and the like are preferably used as promoters for high expression. Any of a wide variety of expression control sequences may be used for the vector in order to express the DNA sequences of the present invention. Useful expression control sequences include, for example, early and late promoters of SV40 or adenovirus, the lac system, the trp system, the TAC or TRC system, T3 and T7 promoters, the major operator and promoter regions of phage lambda, control regions of fd code proteins, promoters of 3-phosphoglycerate kinase or other glycol lyases, promoters of phosphatase, such as Pho5, promoters of yeast alpha-mating systems, and other sequences known to control gene expression of prokaryotic or eukaryotic cells or viruses and various combinations thereof. The T7 promoter may be useful for expressing proteins of the present invention in *E. coli.*

When a nucleic acid sequence is aligned with another nucleic acid sequence based on a functional relationship, it is "operably linked" thereto. This may be gene(s) and control sequence(s) linked in such a way so as to enable gene expression when a suitable molecule (e.g., a transcriptional activator protein) is linked to the control sequence(s). For example, DNA for a pre-sequence or secretory leader is operably linked to DNA for a polypeptide when expressed as a pre-protein involved in the secretion of the polypeptide, a promoter or enhancer is operably linked to a coding sequence when it affects the transcription of the sequence, a ribosome-binding site is operably linked to a coding sequence when it affects the transcription of the sequence, or the ribosome-binding site is operably linked to a coding sequence when positioned to facilitate translation. Generally, "operably linked" means that the linked DNA sequence is in contact therewith, and that a secretory leader is in contact therewith and is present in the reading frame. However, the enhancer need not be in contact therewith. The linkage of these sequences is carried out by ligation (linkage) at convenient restriction enzyme sites. When no such site exists, a synthetic oligonucleotide adapter or a linker according to a conventional method is used.

As used herein, the term "expression vector" commonly refers to a recombinant carrier into which a fragment of heterologous DNA is inserted, and generally means a fragment of double-stranded DNA. Herein, "heterologous DNA" means xenogenous DNA that is not naturally found in the host cell. Once an expression vector is present in a host cell, it can replicate independently of the host chromosomal DNA, and several copies of the vector and inserted (heterologous) DNA thereof can be produced.

As is well known in the art, in order to increase the expression level of a transfected gene in a host cell, the gene should be operably linked to a transcriptional/translational expression control sequence that functions in the selected expression host. Preferably, the expression control sequence and the corresponding gene are included in a single recombinant vector containing both a bacterial selection marker and a replication origin.

The host cell transformed or transfected by the recombinant vector described above constitutes another aspect of the present invention. As used herein, the term "transfection" means introduction of DNA into a host to make the DNA replicable by an extrachromosomal factor or chromosomal integration. As used herein, the term "transformation" means that an expression vector is accommodated by the host cell, regardless of whether or not any coding sequence is actually expressed.

**Example**

Hereinafter, the present invention will be described in more detail with reference to the following examples. However, it will be obvious to those skilled in the art that the following examples are provided only for illustration of the present invention and should not be construed as limiting the scope of the present invention.

**Example 1: Establishment of mouse macular degeneration model**

A macular degeneration mouse model was established using black C57 BL/6 mice. Macular degeneration of mice was induced as follows using a retinal laser.

Each mouse was subjected to general anesthesia through intraperitoneal injection with an animal anesthetic (40 mg/kg zolazepam/tiletamine and 5 mg/kg xylazine), the pupil thereof was expanded with a pupil expander (0.5% tropicamide and 2.5% phenylephrine), and then a circular laser beam having a diameter of 200 $\mu$m (20 mS, 100 mW) was applied to the optic nerve site with a laser having a wavelength of 532 nm to induce choroidal neovascularization (CNV) by laser photocoagulation.

**Example 2: Construction of rAAV2-sVEGFRv-1**

Soluble VEGF receptor-1 variant (sVEGFRv-1) was designed using the human vascular endothelial growth factor receptor (VEGFR) 1 gene (XM_017020485.1, NCBI reference Sequence, NIH).

As shown in FIG. 1A, sVEGFRv-1 of the present invention is a variant which has the same 6 immunoglobulin-like domains having binding sites with VEGF and PIGF and the same 31 N-terminals as FLT-1, and FLT-1s, which is naturally derived from FLT-1, has a tail composed of 6 immunoglobulin-like domains and 37 amino acids different from FLT-1, so the variants have configurations different from each other (Kendall, R.L. and Thomas, K.A. Proc. Natl. Acad. Sci. USA 90, 10705-10709, 1993).

In order to produce pAAV2-sVEGFRv-1, the sVEGFRv-1 spanning nucleotide position 282-2253 (total 1972 bp: SEQ ID NO: 1) was inserted into pAAV-F.IX cis plasmid containing a CMV promoter, an SV40 polyadenylation signal and two ITRs (US Patent No. 6, 093, 392). rAAV2-GFP was produced by inserting the GFP gene into the same plasmid.

The sVEGFRv-1 insertion fragment was prepared through PCR using the following primer pair.

sVEGFRv-1 F1: AAGGTACCGC CACCATGGTCAGCTACTGGGACA (SEQ ID NO: 2)
sVEGFRv-1 R3: CGCTCGAGCTA TCTGATTGTAATTTCTTTCTTCTG (SEQ ID NO: 3)

In order to produce the recombinant rAAV2-sVEGFRv-1 vector and rAAV2-GFP used for gene therapy, AAV rep-cap plasmid DNA (pAAV-R2C2 plasmid, Stratagene Co., USA) which expresses the AAV rep part and cap part and adenovirus helper plasmid (pHelper plasmid, Stratagene Co., USA), in addition to the pAAV-sVEGFRv-1 produced above, is required. The three types of plasmid DNA (pAAV-sVEGFRv-1, pAAV-R2C2 and pHelper) were transfected into HEK293 (human embryonic kidney 293; ATCC CRL-1573) cells, followed by culturing for 72 hours. Then, the HEK293 cells were collected and sonicated, and recombinant AAV (rAAV) particles were repeatedly subjected to CsCl density-gradient centrifugation three times, and portions having an RI (refractive index) of 1.37 to 1.42 g/mL upon the primary measurement and portions having an RI of 1.35 to 1.43 g/mL upon the secondary measurement were collected and purely isolated to obtain a rAAV2-sVEGFRv-1 vector. rAAV2-GFP was obtained in the same manner as above.

**Example 3: Confirmation of VEGFRv-1 expression in cells and tissues treated with rAAV2-sVEGFRv-1**

3-1: Confirmation of sVEGFRv-1 expression in HeLa cells

$1\times10^6$ HeLa cells were treated at MOI of 10,000 with rAAV2-sVEGFR-1 or rAAV2-GFP for 2 days, or were not treated, or were treated with adenovirus 5 at MOI of 5. After the treated cell supernatant was collected, the cells were lysed. 10 basic amino acid sequences of sVEGFR form the binding site of heparin. For Western blotting, sVEGFRv-1 was concentrated in a culture medium using heparin-sepharose beads based on the heparin-binding characteristics of sVEGFR (Biovision, Milpitas, CA). The concentrated protein was developed on an SDS-PAGE gel and transferred to a PVDF membrane, and the protein band was detected using an ECL system. Primary antibodies of sVEGFRv-1 (AF321) and β-actin (YIF-LF-PA0209A) were purchased from R&D systems (Minneapolis, Minnesota) and AbFrontier (Seoul, Korea), respectively. As a result, as can be shown from FIG. 1B, the sVEGFR-1 protein was successfully expressed in rAAV2-sVEGFR-1-treated HeLa cells.

In addition, in order to confirm sVEGFv-1 contained in the cell lysate or cell supernatant, ELISA analysis was performed. For this purpose, a human VEGF R1/Flt-1 Quantikine ELISA Kit (R & D Systems) was used. Analysis was performed according to the manufacturer's manual, and the sVEGFv-1 concentration was calculated using the human VEGF R1 standard provided from the kit.

As a result, as can be seen from FIGS. 1C and 1D, rAAV2-sVEGFRv-1-treated HeLa cells secreted sVEGFRv-1 into an extracellular culture medium at 88.5 ± 11.4%, and the amount of sVEGFRv-1 secreted into the culture medium for 48 hours was 70.01 ± 9.05 ng/$10^6$ cells.

**3-2: Confirmation of sVEGFRv-1 expression in retinal cells**

The expression of sVEGFRv-1 in retinal cells treated with rAAV2-sVEGFRv-1 was confirmed through semi RT-PCR using ARPE-19 cells and retinal tissues of rats.

Human ARPE-19 retinal pigment epithelial cells (ATCC CRL 2302, USA) were cultured at 37°C under 5% $CO_2$ in RPMI medium containing 10% FBS (Invitrogen), 15 mM HEPES (Sigma, St. Louis, MO, USA), GlutaMAX-1 (2 mM), and penicillin (100 IU/ml)/streptomycin (50 μg/ml). The cultured cells were treated at a density of $1x10^4$ virus genomes/cell with the rAAV2-sVEGFRv-1 vector and at a density of 5 virus genomes/cell with the adenovirus serotype 5 vector, and were allowed to stand for 48 hours. Then, the collected cells were subjected to reverse transcription-PCR (RT-PCR) analysis as follows.

ARPE-19 cells treated with rAAV2-sVEGFR-1 or rAAV2-GFP were collected and total RNA was isolated using TRIzol reagent (Invitrogen). 1 μg of the isolated total RNA was subjected to reverse transcription using an MG cDNA synthesis kit (MGmed, Seoul, Korea) to produce cDNA. RT-PCR was conducted using the following primer pair in each sample using the produced cDNA, and the expression of sVEGFR-1 or GFP was identified on a 2% agarose gel to determine whether or not transduction occurred.

β-actin:

Forward: 5'-CACAGAGCCTCGCCTTTGCCGAT (SEQ ID NO: 4)
Reverse: 5'-GGAGCCACACGCAGCTCATTG (SEQ ID NO: 5)

sVEGFR1:

Forward: 5'-TGTCATCGTTTCCAGACCCG (SEQ ID NO: 6)
Reverse: 5'-GGGTGCCAGAACCACTTGAT (SEQ ID NO: 7)

GFP:

Forward: 5'-GGCCGACAAGCAGAAGAAC (SEQ ID NO: 8)
Reverse: 5'-CGCGCTTCTCGTTGGGGTCTT (SEQ ID NO: 9)

In addition, 5 μL of a rAAV2-sVEGFR-1 or rAAV2-GFP vector was injected at a concentration of $5.0 \times 10^{10}$ viral genomes [vg]/mL into the vitreous cavity of the right eye of each rat. After 7 days, the eyeball of the rat treated with rAAV2-sVEGFR-1 or rAAV2-GFP was removed, total RNA was extracted from the eyeball tissues, and RT-PCR was conducted in the same manner as above.

As a result, as shown in FIG. 2, when treated with rAAV2-sVEGFRv-1, sVEGFRv-1 mRNA levels rapidly increased in all of cells (A and C) and tissues (B and D), whereas mRNA levels of GFP increased in cells and tissues treated with rAAV2-GFP as the negative control group.

In order to determine whether or not rAAV2-sVEGFRv-1 has anti-VEGF activity, a migration assay was performed using human umbilical vein endothelial cells (HUVEC) .

The HUVEC cell line (Cambrex Bio Science Walkersville, Inc., USA) was cultured in EGM-2 complete medium (Cat# CC-3162, Cambrex Bio Science) and treated at a MOI of 200,000 with rAAV2-sVEGFR-1 or rAAV2-GFP. After culturing for 48 hours, a scratch was formed along the cells using a cell scraper to induce a wound, followed by washing twice. VEGF (Calbiochem, Cat# 676472, Walkersville, MA) at a final concentration of 10 ng/mL was introduced into a viral-vector-treated sample and an untreated control sample, followed by culturing again to allow the cells to regrow into the wound site. Complete wound closure calculation was conducted using the following equation.

Percentage closure (%) = (cell movement length (mm x 2 x wound length) x 100 / 0.9 mm x length).

As a result, as shown in E and F of FIG. 1, when the scratch wound was formed on HUVEC cells expressing rAAV2-sVEGFRv-1 and then treated with VEGF, cell migration to the wound was reduced. The untreated and control cells cultured in the presence of VEGF grew again into scratch wounds, as expected. In contrast, HUVEC cells transduced with rAAV2-sVEGFRv-1 inhibited the neovascularization effect of VEGF, so that the smallest number of cells migrated to the scratch wound among all cell groups, which ascertains the function of the viral vector. The number of cells that migrated to the scratch wound was $113.4 \pm 17.6$ for rAAV2-VEGFRv-1, $195.8 \pm 19.3$ ($p = 0.00003$) for rAAV2-GFP control vector and $217.2 \pm 21.7$ ($p = 0.00011$) when not treated with a vector ($n = 5$). These results support that rAAV2-sVEGFRv-1 produces a soluble variable VEGF receptor-1 product having the function of inhibiting VEGF activity.

**Example 4: Anti-neovascularization effect of rAAV2-sVEGFRv-1**

14 days after retinal damage induced through laser photocoagulation of the CNV mouse model, a 4:1 mixture of zolazepam/tiletamine (80 mg/kg) and xylazine (10 mg/kg) was intraperitoneally injected into the mouse to perform anesthesia. 0.1M PBS (7.4 pH) was perfused into the heart and 4% paraformaldehyde (PFA) (0.1M PBS) was administered. For the whole mount of the retinal pigment epithelium (RPE), an eyeball was removed. In addition, the eye including the cornea, lens, and retinal nerves was enucleated to produce an eye cup. The cornea and lens were separated from the eyeball, and the eye cup was immersed in an optimal cutting temperature compound (Tissue-Tek, Miles Scientific, Napierville, IL) to produce a 5 μm thick frozen section sample.

Histochemical analysis by immunostaining was performed as follows. The RPE-choroid tissue was cultured from the frozen fragment in mouse anti-CD31 (550274; BD Pharmingen, Inc., San Diego, CA), diluted in PBS containing 1% Triton X-100 (PBST) at 4°C overnight, and the entire mount was immunostained. The result was washed with PBST for 10 minutes three times, reacted with Alexa Fluor 532-conjugated goat anti-mouse (A21270; Thermo Fisher Scientific, Waltham, MA) and rhodamine-conjugated phalloidin (R415; Thermo Fisher Scientific) at room temperature for 2 hours. Whole mounts and frozen sections were examined using fluorescence confocal microscopy (LSM 700, Carl Zeiss Microscopy GmbH, Jena, Germany), and images were captured using ImageJ.

In order to confirm the anti-angiogenic effect of rAAV2-sVEGFRv-1, the intensity of the produced choroidal neovascularization (CNV) was visualized by immunostaining with phalloidin specific for F actin for RPE and fibroblasts and anti-CD31 for endothelial cells (FIG. 3). As a result, as can be seen from FIG. 3, damage to RPE and formation of penetrating new blood vessels were readily observed in untreated control mice and mice intravitreally injected with rAAV-GFP, whereas choroidal neovascularization (CNV) was significantly suppressed in mice injected with rAAV2-sVEGFRv-1 in the vitreous body of the eyeball thereof.

The anti-angiogenic (anti-neovascularization) effect of rAAV2-sVEGFRv-1 was similar to that of bevacizumab, which is widely used in the treatment of wet macular degeneration. The CNV area measured by phalloidin was 1336 ± 186 for the rAAV2-sVEGFRv-1-treated group, 2949 ± 437 (p = 0.0043) for the rAAV2-GFP-treated group, 3075 ± 265 (p = 0.0013) for the untreated group, and 995 ± 234 for the bevacizumab-treated group (n = 3).

**Example 5: Anti-inflammatory effect of rAAV2-sVEGFRv-1**

The diagnosis of wet macular degeneration (AMD) is associated with the penetration and proliferation of inflammatory cells during choroidal neovascularization (CNV). For immunohistochemical evaluation, the presence of inflammatory cells was confirmed using anti-F4/80 (FIG. 4, n = 5) and anti-CD11b (FIG. 5, n = 5) specific for macrophages and leukocytes, respectively, in the retinal section of Example 7.

Frozen sections were stained with anti-CD11b or anti-F4/80 (MCA497GA, Serotec, Oxford, UK) for macrophage, incubating with a diluted primary antibody for overnight, and then washed three times with PBST. The frozen sections were then incubated with a secondary antibody, Alexa Fluor 568 or 488 (Thermo Fisher Scientific) at room temperature for 2 hours and stained with DAPI for nuclear visualization. Whole mounts and frozen sections were examined using fluorescence confocal microscopy (LSM 700, Carl Zeiss Microscopy GmbH, Jena, Germany), and images were captured using ImageJ ZEN (Blue edition).

As a result, as can be seen from FIGS. 4 and 5, both macrophages and leukocytes could be easily detected from both the untreated control group and the rAAV2-GFP-treated group (anti-F4/80: 77.0 ± 9.2 (untreated group) and 82.0 ± 9.0 (rAAV2-GFP-treated group); anti-CD11b: 72.0 ± 7.3 (untreated group) and 75.6 ± 10.5 (rAAV2-GFP-treated group). In contrast, infiltration of inflammatory cells was significantly reduced in the rAAV2-sVEGFRv-1-treated group (anti-F4/80: 35.0 ± 6.9, anti-CD11b: 42.2 ± 8.7). This reduction rate was higher than that of the bevacizumab-treated group (anti-F4/80: 33.2 ± 6.8, anti-CD11b: 49.6 ± 8.8), although the difference was not statistically significant (see M in FIG. 4 and Q in FIG. 5).

**Example 6: Anti-fibrotic effect of rAAV2-sVEGFRv-1**

In order to confirm the anti-fibrotic effect of rAAV2-sVEGFRv-1, transverse retinal sections were immunostained with anti-pan cytokeratin (ab27988; Abcam, San Francisco, CA), which is an index of fibrosis of retinal pigment epithelial cells, in the retinal section of Example 4.

As a result, as can be seen from FIG. 5, retinal fibrosis was easily observed along with CNV in the untreated control group and the rAAV2-GFP-treated mouse-derived retinal section (4353.0 ± 462.0 and 4429.3 ± 671.5 pan cytokeratin-positive region, n = 3, respectively). In contrast, the rAAV2-sVEGFRv-1-treated group (2534.7 ± 703.5, n = 3) exhibited a significant reduction in the number of anti-pan cytokeratin-positive cells, similar to the bevacizumab-treated group (2507.0 ± 585.4, n = 3), which indicates that the rAAV2-sVEGFRv-1-treated group has an inhibitory effect on retinal fibrosis characteristically occurring in wet AMD (FIG. 5R).

**Example 7: Anti-apoptosis effect of rAAV2-sVEGFRv-1**

In order to confirm the apoptosis effect of rAAV2-sVEGFRv-1, TUNEL-positive cells were identified in the retinal section of Example 4.

As a result, as can be seen from FIG. 6, significantly fewer TUNEL-positive cells were observed in samples obtained from mice treated with rAAV2-sVEGFRv-1 and apoptotic cells were easily observed outer nuclear layer (ONL) and CNV in untreated mice or rAAV2-GFP-treated mice (p = 0.001, p = 0.003, n = 3).

In addition, reduction of apoptosis due to treatment with the viral vector rAAV2-sVEGFRv-1 was similar to reduction of apoptosis due to treatment with bevacizumab. The number of TUNEL-positive cells was 16.0 ± 3.6 for the rAAV2-sVEGFRv-1-treated group, 46.0 ± 7.5 for the rAAV2-GFP-treated group, 47.0 ± 5.0 for the untreated control group, and 18.3 ± 2.1 for the bevacizumab-treated group (FIG. 6M).

[Industrial availability]

The present invention is expected to have the effect of treating macular degeneration by inhibiting choroidal neovascularization of macular degeneration patients for several years through only a single administration.

Although specific configurations of the present invention have been described in detail, those skilled in the art will appreciate that preferred embodiments of this description are given for illustrative purposes and should not be construed as limiting the scope of the present invention. Therefore, the substantial scope of the present invention is defined by the accompanying claims and equivalents thereto.

[Sequence Listing Free Text]

An electronic file is attached.

## Claims

1. A recombinant vector comprising a soluble VEGF receptor variant cDNA represented by SEQ ID NO: 1.

2. The recombinant vector according to claim 1, wherein the recombinant vector is adeno-associated virus (AAV).

3. The recombinant vector according to claim 1, wherein the recombinant vector is rAAV2 or another serotype of rAAV.

4. A pharmaceutical composition for treating or preventing macular degeneration comprising a recombinant vector comprising a soluble VEGF receptor variant cDNA.

5. The pharmaceutical composition according to claim 4, wherein the soluble VEGF receptor variant cDNA is represented by SEQ ID NO: 1.

6. The pharmaceutical composition according to claim 4, wherein the recombinant vector is adeno-associated virus (AAV).

7. The pharmaceutical composition according to claim 4, wherein the recombinant vector is rAAV2 or another serotype of rAAV.

Fig. 1

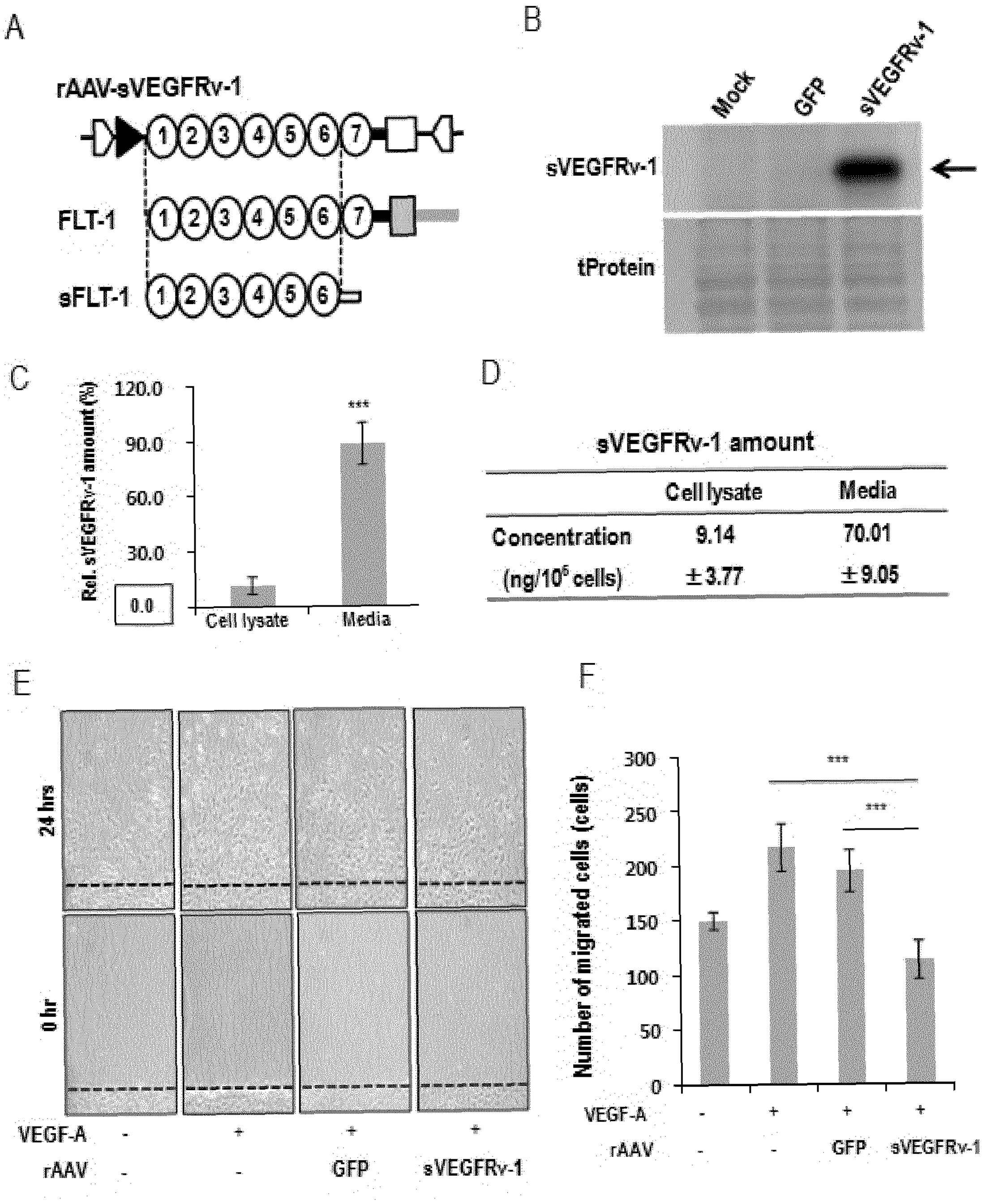
A
rAAV-sVEGFRv-1
FLT-1
sFLT-1
B
Mock
GFP
sVEGFRv-1
sVEGFRv-1
tProtein
C
Rel. sVEGFRv-1 amount (%)
120.0
90.0
60.0
30.0
0.0
***
Cell lysate
Media
D
sVEGFRv-1 amount
Cell lysate
Media
Concentration
(ng/10^6 cells)
9.14
±3.77
70.01
±9.05
E
24 hrs
0 hr
VEGF-A - + + +
rAAV - - GFP sVEGFRv-1
F
Number of migrated cells (cells)
300
250
200
150
100
50
0
***
***
VEGF-A - + + +
rAAV - - GFP sVEGFRv-1

Fig. 2

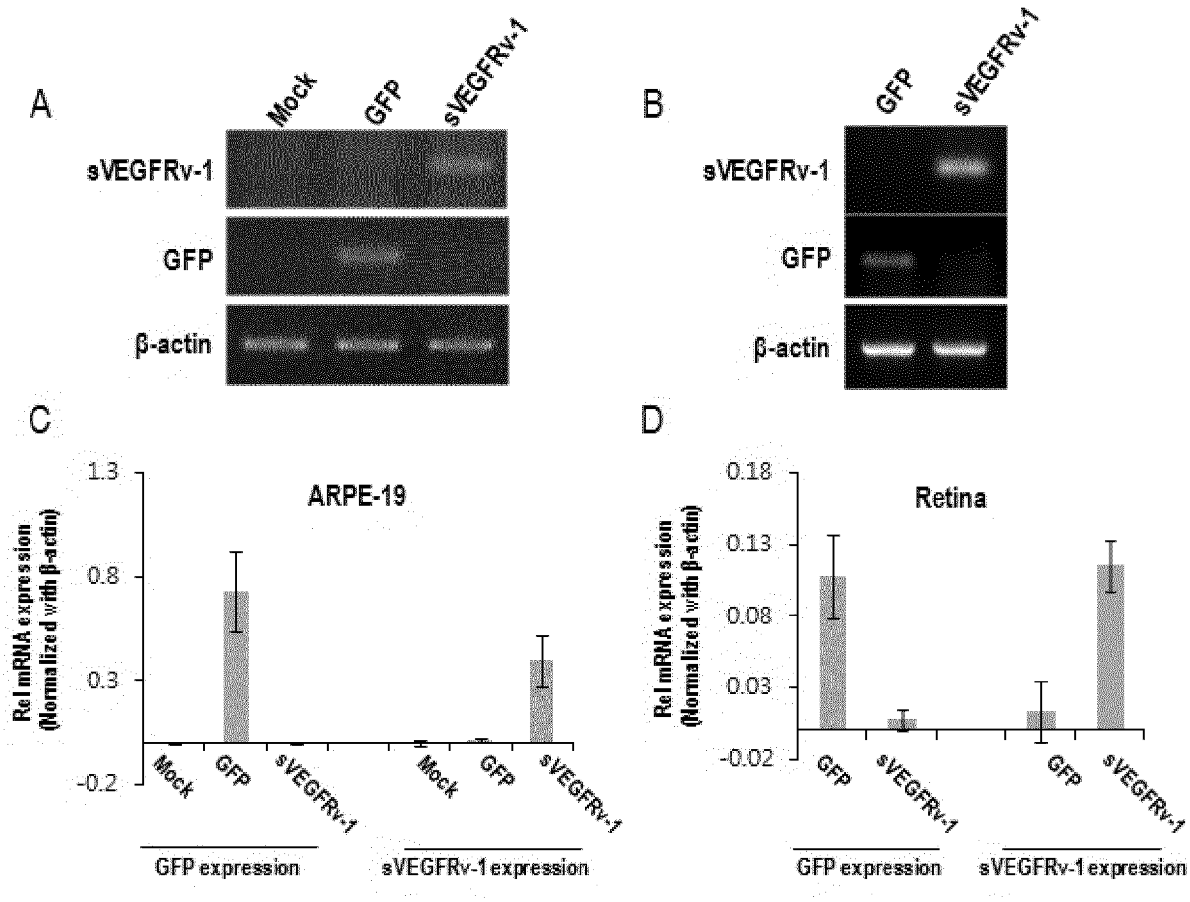
A
Mock
GFP
sVEGFRv-1
sVEGFRv-1
GFP
β-actin
B
GFP
sVEGFRv-1
sVEGFRv-1
GFP
β-actin
C
ARPE-19
Rel mRNA expression (Normalized with β-actin)
1.3
0.8
0.3
-0.2
Mock
GFP
sVEGFRv-1
Mock
GFP
sVEGFRv-1
GFP expression
sVEGFRv-1 expression
D
Retina
Rel mRNA expression (Normalized with β-actin)
0.18
0.13
0.08
0.03
-0.02
GFP
sVEGFRv-1
GFP
sVEGFRv-1
GFP expression
sVEGFRv-1 expression

Fig. 3

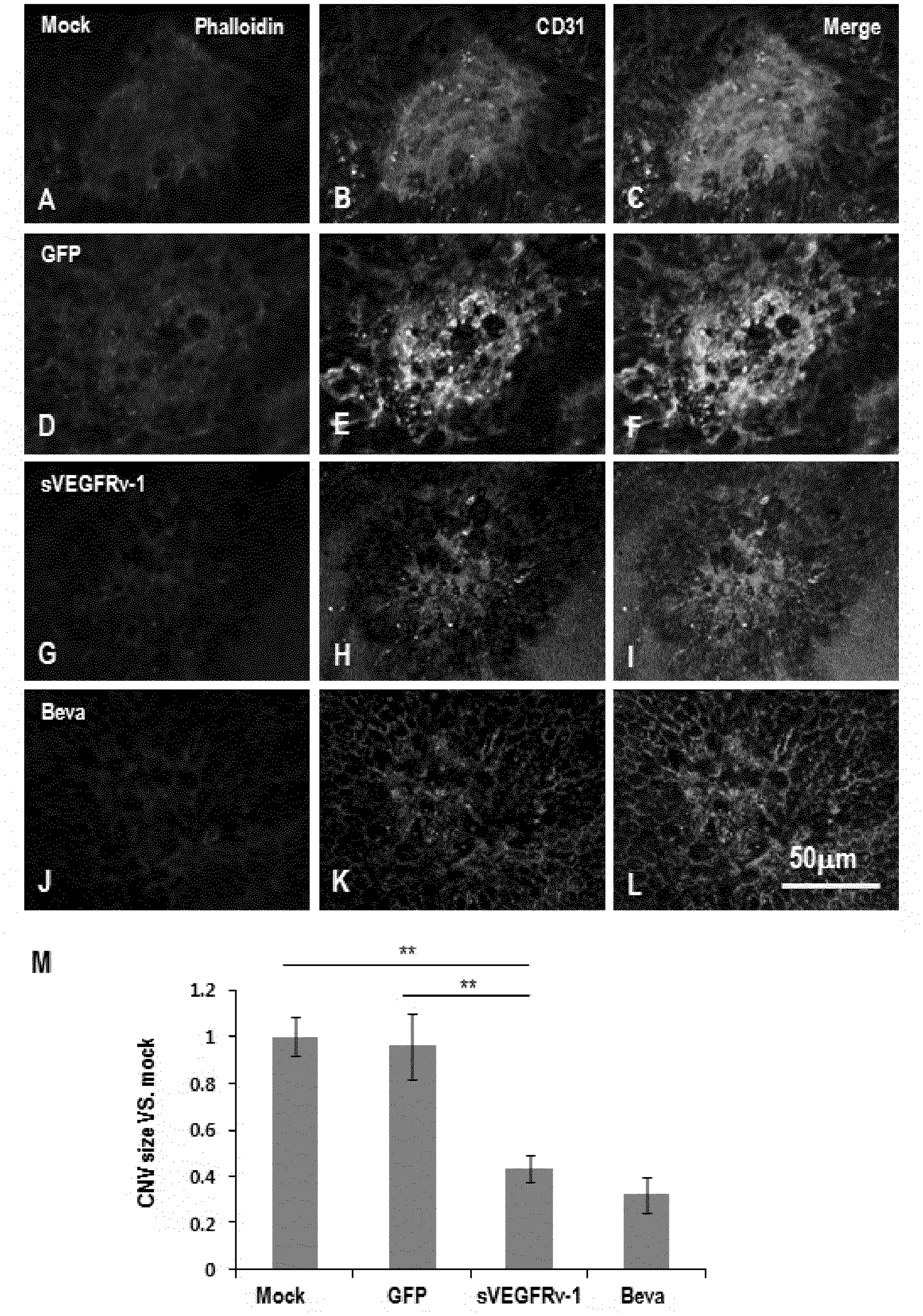
Mock
Phalloidin
CD31
Merge
A
B
C
GFP
D
E
F
sVEGFRv-1
G
H
I
Beva
J
K
L
50μm
M
**
**
1.2
1
0.8
0.6
0.4
0.2
0
CNV size VS. mock
Mock
GFP
sVEGFRv-1
Beva

Fig. 4

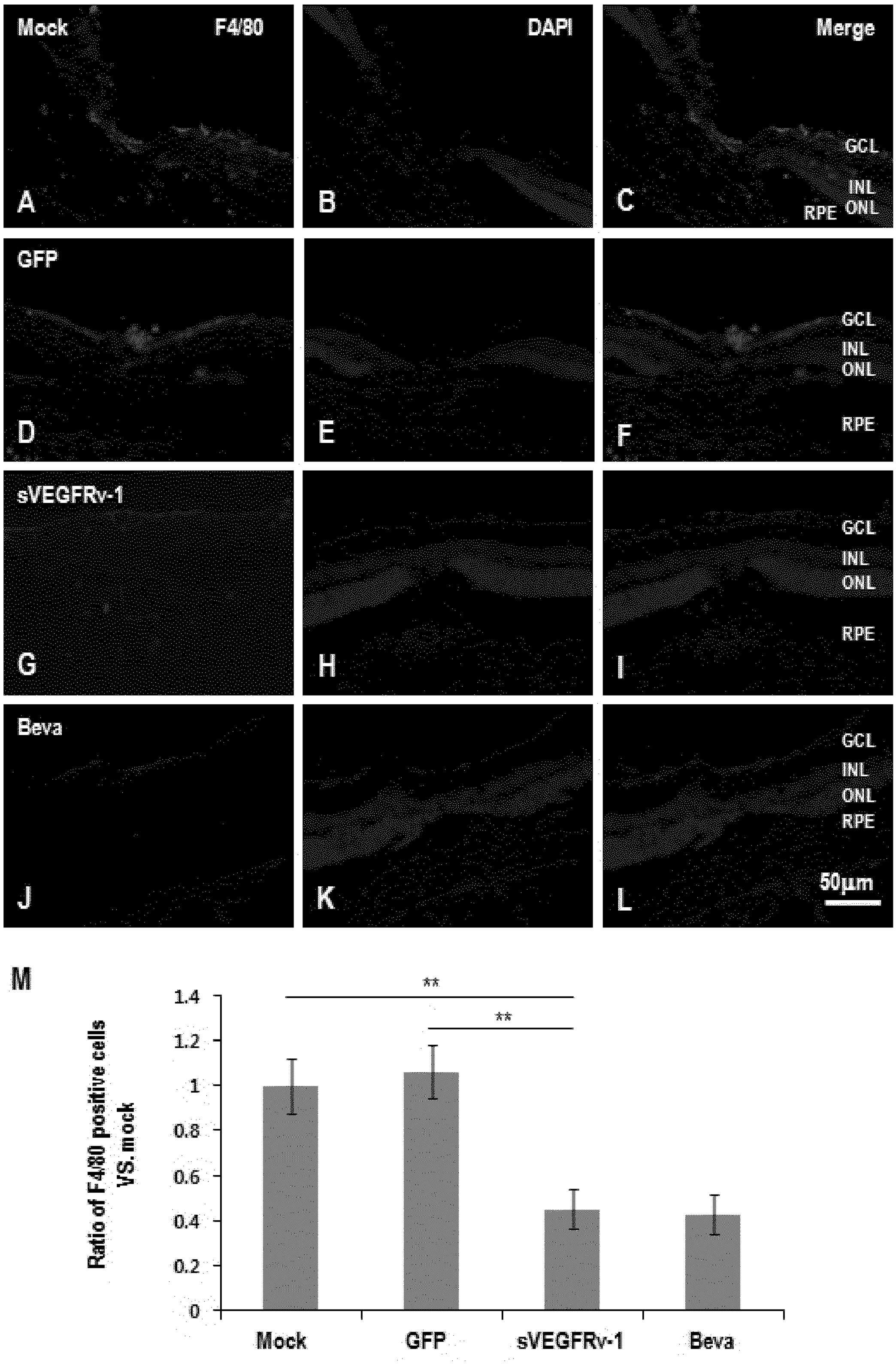
Mock
F4/80
DAPI
Merge
GCL
INL
ONL
RPE
A
B
C
GFP
D
E
F
sVEGFRv-1
G
H
I
Beva
J
K
L
50µm
M
Ratio of F4/80 positive cells VS.mock
1.4
1.2
1
0.8
0.6
0.4
0.2
0
**
**
Mock
GFP
sVEGFRv-1
Beva

Fig. 5

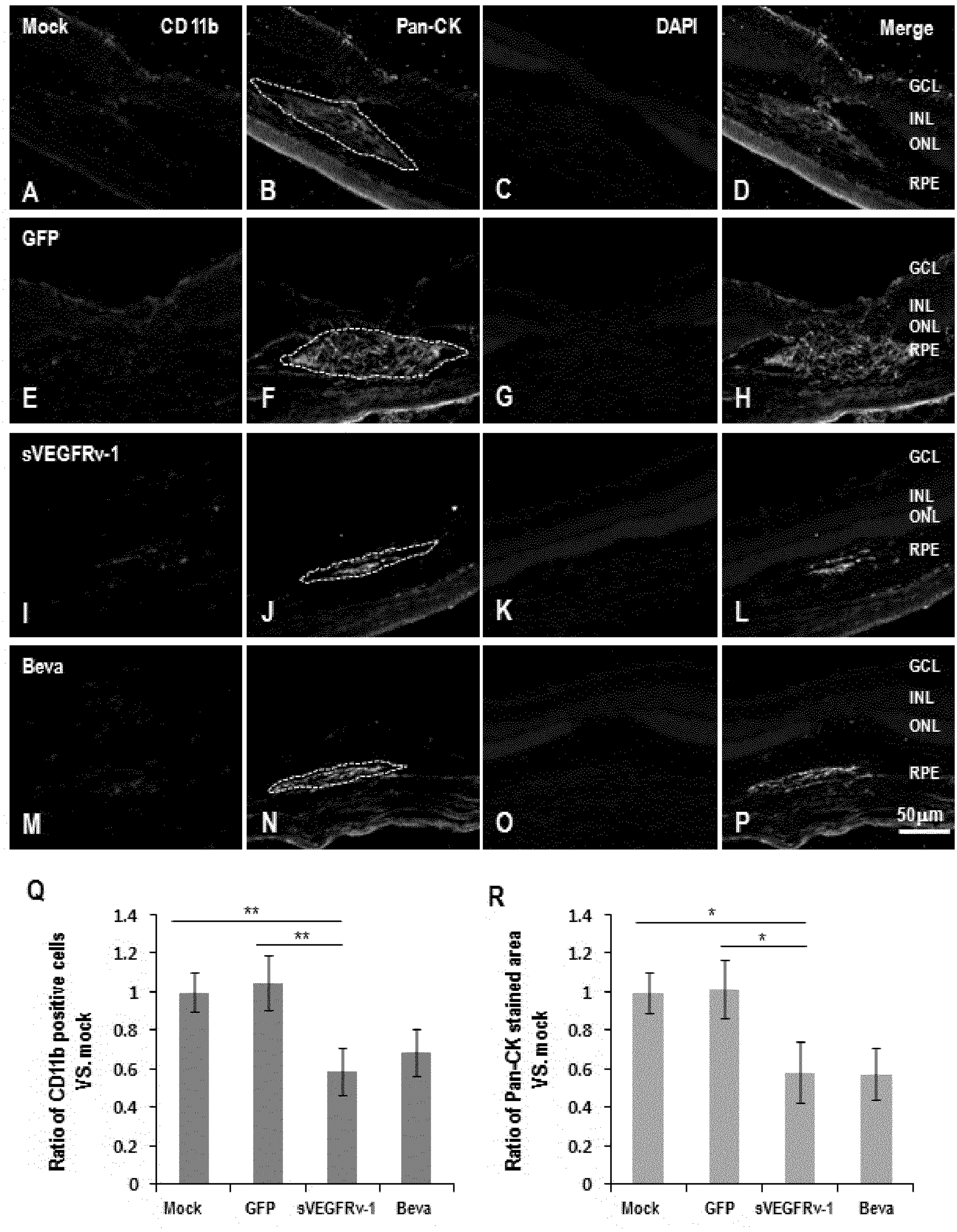
Mock
CD11b
Pan-CK
DAPI
Merge
GCL
INL
ONL
RPE
A
B
C
D
GFP
E
F
G
H
sVEGFRv-1
I
J
K
L
Beva
M
N
O
P
50μm
Q
Ratio of CD11b positive cells VS. mock
1.4
1.2
1
0.8
0.6
0.4
0.2
0
**
**
Mock
GFP
sVEGFRv-1
Beva
R
Ratio of Pan-CK stained area VS. mock
*
*

Fig. 6

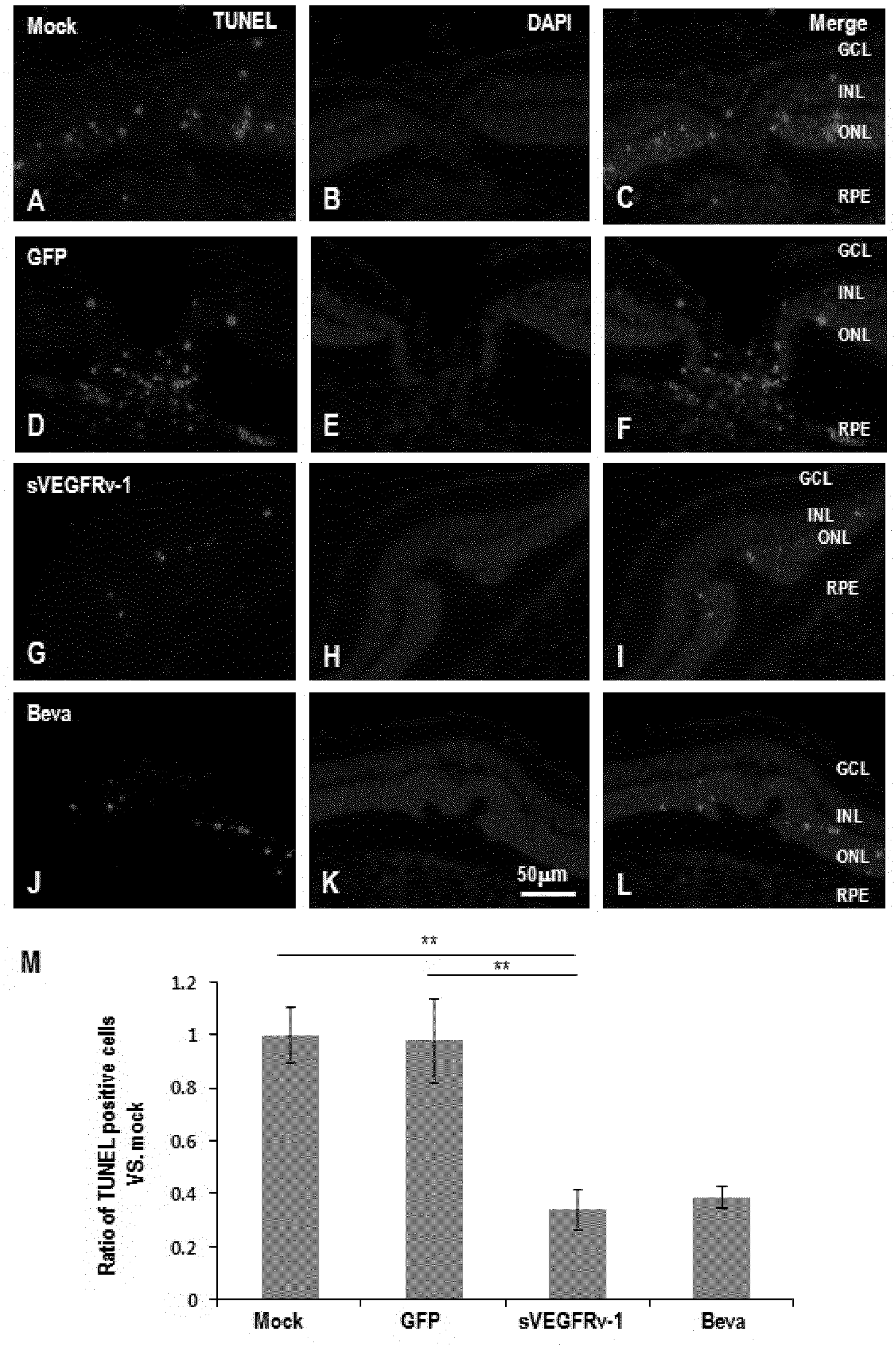
Mock
TUNEL
DAPI
Merge
GCL
INL
ONL
RPE
A
B
C
GFP
D
E
F
sVEGFRv-1
G
H
I
Beva
J
K
L
50μm
M
**
**
Ratio of TUNEL positive cells VS. mock
1.2
1
0.8
0.6
0.4
0.2
0
Mock
GFP
sVEGFRv-1
Beva

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6093392 A


### Non-patent literature cited in the description


- **WONG WL et al.** *Lancet Glob. Health,* 2014, vol. 2, e106
- **NOWAK J.Z.** *Pharmacol. Rep.,* 2006, vol. 58, 353
- **KENDALL, R.L. ; THOMAS, K.A.** *Proc. Natl. Acad. Sci. USA,* 1993, vol. 90, 10705-10709
- **NEUMANN et al.** *EMBO J.,* 1982, vol. 1, 841

<110> KRgeneBIO Co., Ltd.

<120> Pharmaceutical Composition for Treating Macular Degeneration Containing AAV Including cDNA of Soluble VEGFR Variant

<130> PP-B2079

<150> 10-2017-0140190
<151> 2017-10-26

<150> KR 10-2018-0112734
<151> 2018-09-20

<160> 9

<170> KopatentIn 2.0

<210> 1
<211> 1972
<212> DNA
<213> Artificial Sequence

<220>
<223> sVEGFRv-1

<400> 1
caccatggtc agctactggg acaccggggt cctgctgtgc gcgctgctca gctgtctgct 60

tctcacagga tctagttcag gttcaaaatt aaaagatcct gaactgagtt taaaaggcac 120

ccagcacatc atgcaagcag gccagacact gcatctccaa tgcagggggg aagcagccca 180

taaatggtct ttgcctgaaa tggtgagtaa ggaaagcgaa aggctgagca taactaaatc 240

tgcctgtgga agaaatggca aacaattctg cagtacttta accttgaaca cagctcaagc 300

aaaccacact ggcttctaca gctgcaaata tctagctgta cctacttcaa agaagaagga 360

aacagaatct gcaatctata tatttattag tgatacaggt agacctttcg tagagatgta 420

cagtgaaatc cccgaaatta tacacatgac tgaaggaagg gagctcgtca ttccctgccg 480

ggttacgtca cctaacatca ctgttacttt aaaaaagttt ccacttgaca ctttgatccc 540

tgatggaaaa cgcataatct gggacagtag aaagggcttc atcatatcaa atgcaacgta 600

caaagaaata gggcttctga cctgtgaagc aacagtcaat gggcatttgt ataagacaaa 660

ctatctcaca catcgacaaa ccaatacaat catagatgtc caaataagca caccacgccc 720

agtcaaatta cttagaggcc atactcttgt cctcaattgt actgctacca ctcccttgaa 780

cacgagagtt caaatgacct ggagttaccc tgatgaaaaa aataagagag cttccgtaag 840

gcgacgaatt gaccaaagca attcccatgc caacatattc tacagtgttc ttactattga 900

caaaatgcag aacaaagaca aaggacttta tacttgtcgt gtaaggagtg gaccatcatt 960

caaatctgtt aacacctcag tgcatatata tgataaagca ttcatcactg tgaaacatcg 1020

aaaacagcag gtgcttgaaa ccgtagctgg caagcggtct taccggctct ctatgaaagt 1080

gaaggcattt ccctcgccgg aagttgtatg gttaaaagat gggttacctg cgactgagaa 1140

atctgctcgc tatttgactc gtggctactc gttaattatc aaggacgtaa ctgaagagga 1200

tgcagggaat tatacaatct tgctgagcat aaaacagtca aatgtgttta aaaacctcac 1260

tgccactcta attgtcaatg tgaaacccca gatttacgaa aaggccgtgt catcgtttcc 1320

agacccggct ctctacccac tgggcagcag acaaatcctg acttgtaccg catatggtat 1380

ccctcaacct acaatcaagt ggttctggca cccctgtaac cataatcatt ccgaagcaag 1440

gtgtgacttt tgttccaata atgaagagtc ctttatcctg gatgctgaca gcaacatggg 1500

aaacagaatt gagagcatca ctcagcgcat ggcaataata gaaggaaaga ataagatggc 1560

tagcaccttg gttgtggctg actctagaat ttctggaatc tacatttgca tagcttccaa 1620

taaagttggg actgtgggaa gaaacataag cttttatatc acagatgtgc caaatgggtt 1680

tcatgttaac ttggaaaaaa tgccgacgga aggagaggac ctgaaactgt cttgcacagt 1740

taacaagttc ttatacagag acgttacttg gattttactg cggacagtta ataacagaac 1800

aatgcactac agtattagca agcaaaaaat ggccatcact aaggagcact ccatcactct 1860

taatcttacc atcatgaatg tttccctgca agattcaggc acctatgcct gcagagccag 1920

gaatgtatac acaggggaag aaatcctcca gaagaaagaa attacaatca ga 1972

<210> 2
<211> 33
<212> DNA
<213> Artificial Sequence

<220>
<223> primer

<400> 2
aaggtaccgc caccatggtc agctactggg aca 33

<210> 3
<211> 35
<212> DNA
<213> Artificial Sequence

<220>
<223> primer

<400> 3
cgctcgagct atctgattgt aatttctttc ttctg 35

<210> 4
<211> 23
<212> DNA
<213> Artificial Sequence

<220>

<223> primer

<400> 4
cacagagcct cgcctttgcc gat 23

<210> 5
<211> 21
<212> DNA
<213> Artificial Sequence

<220>
<223> primer

<400> 5
ggagccacac gcagctcatt g 21

<210> 6
<211> 20
<212> DNA
<213> Artificial Sequence

<220>
<223> primer

<400> 6
tgtcatcgtt tccagacccg 20

<210> 7
<211> 20
<212> DNA
<213> Artificial Sequence

<220>
<223> primer

<400> 7
gggtgccaga accacttgat 20

<210> 8
<211> 19
<212> DNA
<213> Artificial Sequence

<220>
<223> primer

<400> 8
ggccgacaag cagaagaac 19

<210> 9
<211> 21
<212> DNA
<213> Artificial Sequence

```
<220>
<223>    primer

<400>    9
cgcgcttctc gttggggtct t                                                21
```